# EUROPEAN PATENT SPECIFICATION

(11) **EP 3 193 629 B1**
(45) Date of publication and mention of the grant of the patent: **25.01.2023**
(21) Application number: 15836374.7
(22) Date of filing: 24.08.2015
(51) Int. Cl.: A23K 50/30, A23K 20/189, A61K 38/48, A23K 10/00, A61P 1/12

(54) **ANTI-DIARRHEA FORMULATION WHICH AVOIDS ANTIMICROBIAL RESISTANCE**
FORMULIERUNG GEGEN DURCHFALL MIT VERHINDERUNG VON ANTIMIKROBIELLER RESISTENZ
FORMULATION ANTI-DIARRHÉIQUE ÉVITANT LA RÉSISTANCE AUX ANTIMICROBIENS

(30) Priority: 25.08.2014 US 201462041175 P
(43) Date of publication of application: 26.07.2017
(73) Proprietor: Anatara Lifesciences Limited, Brisbane, QLD 4120 (AU)
(72) Inventor: MYNOTT, Tracey L., QLD 4005 (AU); WALSH, John, Sydney, New South Wales 2096 (AU)
(74) Representative: Haseltine Lake Kempner LLP
(86) International application number: PCT/US2015/046509
(87) International publication number: WO 2016/032944

(56) References cited:
- WO-A1-88/01506
- WO-A1-2008/049437
- WO-A1-2009/009879
- CN-A- 1 548 154
- US-A- 5 484 609
- US-A- 6 080 403
- US-A1- 2006 052 438
- CHANDLER ET AL.: 'Bromelain protects piglets from diarrhea caused by oral challenge with K88 positive enterotoxigenic Escherichia coli' GUT vol. 43, no. 2, 01 January 1998, pages 196 - 202, XP002464839
- GASPANI ET AL.: 'In vivo and in vitro Effects of Bromelain on PGE2 and SP Concentrations in the Inflammatory Exudate in Rats' PHARMACOLOGY vol. 65, 01 May 2002, pages 83 - 86, XP009500538
- MYNOTT ET AL.: 'Bromelain Prevents Secretion Caused by Vibrio cholerae and Escherichia coli Enterotoxins in Rabbit Ileum In Vitro' GASTROENTEROLOGY vol. 113, no. 1, 01 July 1997, pages 175 - 184, XP005688534 DOI: 10.1016/S0016-5085(97)70093-3
- MYNOTT ET AL.: 'Oral administration of protease inhibits enterotoxigenic Escherichia coli receptor activity in piglet small intestine' GUT vol. 38, no. 1, 01 January 1996, pages 28 - 32, XP055411985 DOI: 10.1136/GUT.38.1.28
- FAIBROTHER ET AL.: 'Escherichia coli in postweaning diarrhea in pigs: an update on bacterial types, pathogenesis, and prevention strategies' ANIMAL HEALTH RESEARCH REVIEWS vol. 6, no. 1, 01 June 2005, pages 17 - 39, XP008176046 DOI: 10.1079/AHR2005105

## Description

### Background:

Diarrhea is a problem in pigs and people. Scour (diarrhea) in piglets is one of the most common problems in pig farming affecting hundreds of millions of the 1,600 million piglets born globally each year. It can result in reduced weight gain, high cost of treatment, and frequent mortality. Piglets set back in health at an early age, tend to remain at a weight and performance disadvantage in later life. Therefore, scours not only has a detrimental impact on piglet health, but on farm profitability.

Diarrhea is also a significant problem in humans. It is the second cause of death in the developing world, killing more than 1.5 million children annually and is a leading cause of malnutrition. About 4 billion cases of diarrhea are estimated to occur every year among children under five years. Diarrhea is also a significant problem for Traveller's to developing areas, affecting 40 million people annually, and is a major problem for military personnel during operations.

There are many different causes of diarrhea. Enterotoxigenic Escherichia coli (ETEC) is one of the most common causes of scour in un-weaned and just-weaned piglets. Other important pathogens include rotavirus and coccidiosis caused by the protozoan Isospora suis and affects mainly piglets in the first three weeks of life. High mortality due to coccidiosis is observed in co-infections with ETEC.

ETEC is also one of the most common causes of diarrhea in young children in developing countries, and is associated with a higher risk of death than other diarrhea-causing pathogens. ETEC are also the most prevalent cause of diarrhea in Traveler's to developing areas with attack rates as high as 70% in some instances. Other important pathogens affecting children under five in developing countries include rotavirus, Cryptosporidium, Shigella, Aeromonas, Vibrio cholerae O1 and Campylobacter. Enteroaggregative E. coli (EAEC), Shigella, Campylobacter, Salmonella, Aeromonas, Plesiomonas, and noncholera Vibrios are other important causes of Traveler's Diarrhea.

These same above pathogens also cause diarrhea in the USA, as well as enterohaemorrhagic E. coli (EHEC, or Shiga toxin-producing E. coli, STEC), Listeria, Yersinia, Cyclospora, Giardia, calciviruses, and other enteric viruses. Clostridium difficile is the major cause of hospital acquired infections. Collectively, these agents are responsible for greater than 200 million cases of diarrhea in the USA each year. In many cases, an infectious agent is not always responsible. For example, in HIV affected patients and cancer patients, diarrhea may result as a chronic side effect of chemotherapy. Diarrhea may also be a result of hormonal imbalances induced by endocrine tumours, diabetes or chronic inflammation, as in patients with inflammatory bowel disease.

All diarrhea-causing pathogens differ markedly in the clinical syndromes they induce, mechanisms of pathogenesis, virulence and epidemiology. Some pathogens, such as ETEC, are non-invasive and induce diarrhea by attaching via specific adherence factors to enterocyte receptors on the small intestine and by the production of enterotoxins. Other pathogens, such as Shigella, are invasive and alter intestinal barrier function and induce diarrhea by inducing inflammation or loss of absorptive surface and malabsorption. This diversity poses an enormous task for researchers who are attempting to design simple and effective prophylaxis or treatment against all causes of diarrhea.

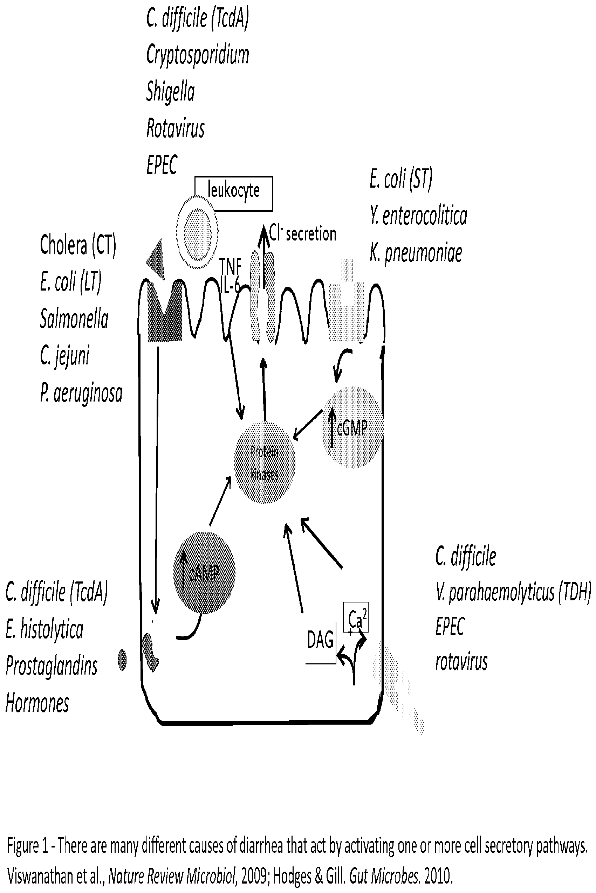

Despite the diversity of pathogens and multitude of factors that induce diarrhea, bacterial toxins or inflammatory mediators induce the most common cause of diarrhea. Toxins typically trigger signaling molecules such as cyclic AMP, cyclic GMP or intracellular Ca²⁺, which, in turn activate intestinal chloride (Cl⁻) channels leading to an increase in secretion of Cl⁻ and consequently fluid secretion. When the level of fluid secretion increases beyond the ability of the colon to reabsorb water and electrolytes lost from the small intestine, diarrhea results that can lead to severe dehydration and eventual death. Chemotherapeutic agents and inflammatory agents also induce diarrhea by activating these signaling molecules. See Figure 1. Agents that target these cyclic nucleotide or calcium signaling pathways would be expected to be anti-secretory agents, and hence effective broad spectrum anti-diarrhea drugs.

Ingestion of pathogenic bacteria such as *Escherichia coli* can cause diarrhea in humans and scour in swine. Scour is of particular concern in modern swine farming, which often entails dense or crowded growing conditions.

Antibiotics are an effective means to treat diarrhea in humans and scour in swine. In farming, antibiotics may also be routinely added to animal feed to prophylactically prevent scour. Regular and widespread antibiotic use, however, has allowed antibiotic-resistant strains of pathogenic bacteria to proliferate.

Thus, there is a need in the art for a way to treat and prevent diarrhea in humans and scour in swine without encouraging the further proliferation of antibiotic-resistant strains of pathogenic bacteria.

Document CN 1548154 discloses the treatment of diarrhea in mammals by Bromelain using compositions that may comprise citric acid and carboxymethylcellulose, among others.

Document WO 2008/049437 discloses the use of compositions comprising lignin and optionally bromelain as growth promoters in piglets.

I found a way. My solution does not kill pathogenic bacteria; it is thus not an "anti-biotic" in the conventional sense of the word, and does not selectively favor the proliferation of antibiotic-resistant bacteria. Rather, my solution merely prevents pathogenic bacteria from adhering to the lining of the gastrointestinal tract. Unable to adhere to the lining of the gastrointestinal tract, the pathogenic bacteria pass through the host animal's gastrointestinal tract unharmed and are eliminated in host animal's stool. My solution also works against other diarrhea causing microbes, such as viruses and parasites. It does this by blocking the invasion and secretory pathways within intestinal cells, and not by trying to kill or target the diarrhea causing microbe. My solution is not poisonous and produces no poisonous metabolite or residue; it thus is suitable for use in humans and in food animals such as swine, cattle and poultry, and in aquaculture (for example, farmed fish and shellfish). It is also useful for companion animals such as dogs and cats, ornamental fish etc. I use the term "veterinary" in the appended claims to encompass food animals, companion animals and fish. Further, it may be synthesized using standard industrial polypeptide synthetic techniques such as Fmoc resin synthesis, but may alternatively also be manufactured from certain botanical extracts.

### Brief Description of the Figures:

Figure 1 diagrams several possible causes of diarrhea.
Figure 2 diagrams three possible mechanisms of action of my formula.
Figure 3 charts the number of post-weaning piglets with scour over time after a single administration of my formula, compared to non-treated piglets.
Figure 4 charts Total Clinical Score of post-weaning piglets over time after a single administration of my formula, compared to non-treated piglets.

### Detailed Description:

My invention is defined by the claims. It basically relates to an oral veterinary formulation comprising an anti-diarrhea effective amount of bromelain and an emulsifier comprising lecithin in an amount effective to improve the solubility of said bromelain in water, and further comprises citric acid. In a particular embodiment, it entails formulating an aqueous oral suspension of bromelain, BLANOSE^{®} sodium carboxymethyl cellulose, citric acid anhydrous, EPIKURON^{®} 135F lecithin oil and ethylenedinitrilotetraacetic acid, disodium salt dihydrate ("EDTA"). For example, a suitable formulation is shown in Table 1.

**Table 1**

| Material | Quantity per Unit Dose | Percent (w/w) |
|---|---|---|
| Bromelain q.s | 62.5 - 88.4 mg | 53.1 - 74.7 |
| BLANOSE^{®} sodium carboxymethyl cellulose | 25.9 - 42.4 mg | 22 - 36 |
| citric acid anhydrous | 4.7 - 14.96 mg | 4 - 12.7 |
| EPIKURON^{®} 135F lecithin oil | 3.5 - 9.3 mg | 3 - 7.9 |
| ethylenedinitrilotetraacetic acid, disodium salt dihydrate | 0.8 - 1.9 mg | 0.7 - 1.6 |
| Total | 97.4 - 156.96 mg | 100.0 |

This formulation produces a free-flowing powder. It may be suspended in 2 mL of water for oral administration to suckling piglets to prevent E. coli, or other microbe induced diarrhea ("scours"). The amount used may be increased when administering to larger animals or to humans. I now provide further detail on each of these components.

### Bromelain

Bromelain is the collective name for a crude proteolytic extract obtained from the pineapple plant (*Ananas comosus*). Two forms of bromelain are known; fruit bromelain obtained from fresh pineapple fruit, and stem bromelain obtained from the stem of the plant. The main commercial source of bromelain is stem bromelain, and the terms "bromelain" and "stem bromelain" are used interchangeably.

Bromelain is prepared from the stems after the fruit is harvested. The stem is peeled, crushed and pressed to obtain a juice containing the soluble bromelain components. Further processing includes concentration, filtration and drying of the pressed juice to get a final white-yellow or tan dry powder. The resultant bromelain extract is a mixture of protein-digesting enzymes-called proteolytic enzymes or proteases-and several other substances in smaller quantities, such as peroxidases, acid phosphatases, certain protease inhibitors, and calcium.

The major proteolytic enzyme within bromelain is a protease called stem bromelain, CAS 37189-34-7 (EC 3.4.22.32), while the major protease within the fruit bromelain extract is called fruit bromelain (EC 3.4.22.33). These proteases enzymes are referred to as sulfhydryl proteases, since a free sulfhydryl group of a cysteine side-chain is required for function. Stem bromelain has a broad specificity for cleavage of proteins, and has a strong preference for Z-Arg-Arg-|-NHMec among small molecule substrates. Fruit bromelain, has a strong preference for Bz-Phe-Val-Arg-|-NHMec.

Unless otherwise qualified (e.g. "fruit bromelain"), I use the term "bromelain" here to refer to the crude extract from pineapple stems, and "stem bromelain protease" to describe the main protease.

Pineapples have a long tradition as a medicinal plant among the natives of South and Central America. The first isolation of bromelain was recorded by the Venezuelan chemist Vicente Marcano in 1891 from the fruit of pineapple. In 1892, Russell Henry Chittenden, assisted by Elliott P. Joslin and Frank Sherman Meara, investigated the matter fully, and called it 'bromelin'. Later, the term "bromelain" was introduced, and originally the term was applied to any protease from any member of the plant family Bromeliaceae.

Bromelain has a long history of folk and modern medicinal use and continues to be explored as a potential healing agent in alternative medicine. It is also widely accepted as a phytotherapeutical drug. Bromelain was first introduced as a therapeutic supplement in 1957. First, research on bromelain was conducted in Hawaii, but more recently has been conducted in countries in Asia, Europe, and Latin America. Recently, researchers in Germany have taken a great interest in bromelain research. Currently, bromelain is the thirteenth most widely used herbal medicine in Germany.

Some of the therapeutic benefits of bromelain are reversible inhibition of platelet aggregation, reversible inhibition of angina pectoris, reversible inhibition of bronchitis and sinusitis, treating surgical traumas, thrombophlebitis, and pyelonephritis. It can also be used after surgery or injury to reduce swelling (inflammation), especially of the nose and sinuses. It is also used for preventing muscle soreness after intense exercise. Bromelain also has been reported to interfere with the growth of tumor cells and slow blood clotting. *See* www.nlm.nih.gov/medlineplus/druginfo/natural/895.html. Bromelain is also used for hay fever, treating a bowel condition that includes swelling and ulcers (ulcerative colitis), removing dead and damaged tissue after burns (debridement), preventing the collection of water in the lung (pulmonary edema), relaxing muscles, stimulating muscle contractions, improving the absorption of antibiotics, preventing cancer, shortening labor, and helping the body get rid of fat. In food preparation, bromelain is used as a meat tenderizer, and to clarify beer.

Systemic enzyme therapy (consisting of combinations of proteolytic enzymes such as bromelain, trypsin, chymotrypsin, and papain) has been investigated in Europe for the treatment of breast, colorectal, and plasmacytoma cancer patients. In mice with experimental colitis, six months of dietary bromelain from pineapple stem or from fresh juice decreased the severity of colonic inflammation and reduced the number of cancerous lesions in the colon.

Bromelain supplements, when taken with other medications (Amoxicillin, antibiotics, anticoagulant/antiplatelet drugs), may increase the risk associated with heart rate, blood clotting and bleeding post-surgery.

Bromelain's anti-metastatic and anti-inflammatory activities are apparently independent of its proteolytic activity. Although poorly understood, the diverse biological effects of bromelain seem to depend on its ability to traverse the membrane barrier, a very unusual property of this compound.

As a potential anti-inflammatory agent, bromelain may be useful for treating arthritis, but has neither been confirmed in human studies for this use, nor is it approved with a health claim for such an effect by the Food and Drug Administration or European Food Safety Authority. The *Natural Medicines Comprehensive Database* suggests that bromelain, when used in conjunction with trypsin (a different protease) and rutin (a substance found in buckwheat) is as effective as some prescription analgesics in the management of osteoarthritis. A product (WOBENZYME^{™}) that combines bromelain with trypsin and rutin is available commercially and seems to reduce pain and improve knee function in people with osteoarthritis. However, the National Institutes of Health notes, "There isn't enough scientific evidence to determine whether or not bromelain is effective for any of its other uses." *See id.* Bromelain is also available in some countries as a product under the name ANANASE^{™}.

Bromelain has not been scientifically proven to be effective in any other diseases and it has not been licensed by the Food and Drug Administration for the treatment of any other disorder.

Bromelain is produced in Thailand, Taiwan, and other tropical parts of the world where pineapples are grown.

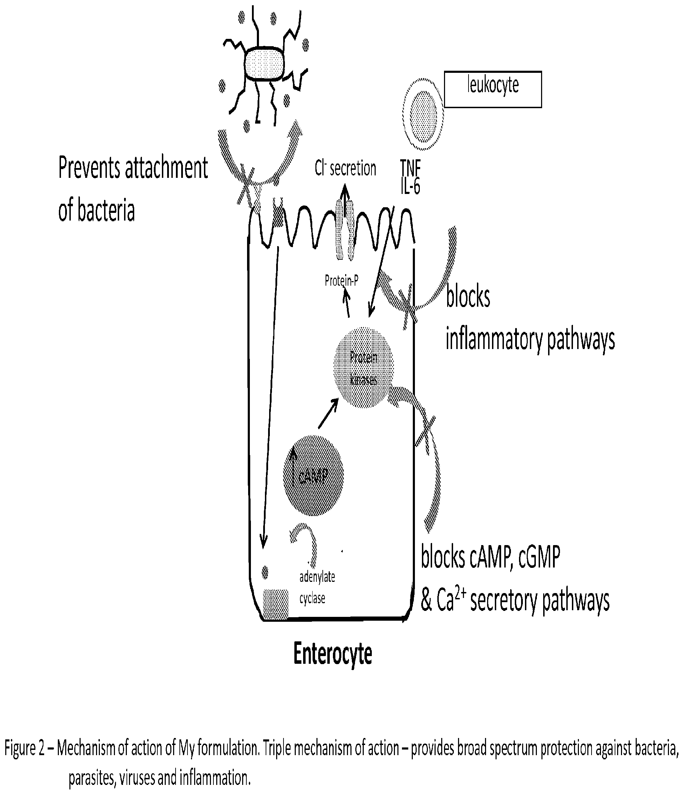

Bromelain has shown promise of an effective, broad spectrum anti-diarrhea drug, as it targets the underlying cause of diarrhea, the inflammatory and secretory pathways. Bromelain has a triple mechanism of action. See Figure 2. First, it prevents the attachment of bacteria to the small intestine thereby preventing their colonization. Secondly, it prevents and reverses the action of bacterial toxins, and inflammatory mediators, the underlying cause of excessive fluid secretion and diarrhea. It does this by blocking enterotoxin and inflammatory mediator-induced cyclic AMP, cyclic GMP and Ca²⁺ intracellular signaling pathways that induce intestinal fluid secretion and secretory diarrhea. Thirdly, bromelain also inhibits inflammation by reducing the production of, and action of pro-inflammatory cytokines, including TNFα, IFNγ, and IL-6 by preventing activation of the ERK-2, JNK and p38 mitogen activated protein (MAP) kinase pathways. These pathways and pro-inflammatory cytokines play a key role in the intestinal barrier dysfunction induced by Shigella, Salmonella, and Clostridium difficile, and in chronic inflammation, such as in patients with inflammatory bowel disease (IBD).

Because bromelain acts on the underlying mechanisms of diarrhea, unlike antibiotics and vaccines that only target specific types of pathogen, bromelain will be effective against a range of different causes of diarrhea. Also because bromelain is not an antibiotic and does not target the pathogens, it should not contribute to the growing problem of antibiotic-resistance, a serious global health problem.

The use of my formulation prevents inflammation at weaning, improving gut health, and increasing feed intake. Weaning is a critical period in a piglet's life. It must cope with separation from the sow, the transition from highly digestible milk to a less digestible and more complex solid feed, a new environment, movement and separation from littermates, and exposure to unfamiliar pigs. These stressors can lead to reduced feed intake and reduced piglet growth.

Additionally, the newly-weaned pigs' immune and digestive systems are still maturing, making the piglet more susceptible to antigenic challenges (nutritional or microbial), which can lead to inflammatory responses. Inflammation induces a negative impact on the digestive and absorptive capabilities of the gut, and overall gut health creating an opportunity for an animal to become more susceptible to pathogens. An animal whose reduced feed intake is poor at the time of pathogen exposure will become sick.

The absence of feed in a piglet's stomach results in a microbial imbalance, leading to higher occurrences of diseases. Thus, this short weaning phase in a pig's life can have far-reaching consequences, negatively affecting the pig's entire rearing period.

The anti-inflammatory activity of bromelain reduces gut inflammation, and protects piglets from disease, as well as increases piglet food intake during the post-weaning period.

### BLANOSE^{®} sodium carboxymethyl cellulose

Cellulose for industrial use is mainly obtained from wood pulp and cotton. The kraft process is used to separate cellulose from lignin, another major component of plant matter. Cellulose has no taste, is odorless, is hydrophilic with the contact angle of 20-30, is insoluble in water and most organic solvents, is chiral and is biodegradable. It can be broken down chemically into its glucose units by treating it with concentrated acids at high temperature.

Cellulose is derived from D-glucose units, which condense through β(1→4) -glycosidic bonds. This linkage motif contrasts with that for α(1→4) -glycosidic bonds present in starch, glycogen, and other carbohydrates. Cellulose is a straight chain polymer: unlike starch, no coiling or branching occurs, and the molecule adopts an extended and rather stiff rod-like conformation, aided by the equatorial conformation of the glucose residues. The multiple hydroxyl groups on the glucose from one chain form hydrogen bonds with oxygen atoms on the same or on a neighbor chain, holding the chains firmly together side-by-side and forming *microfibrils* with high tensile strength. This confers tensile strength in cell walls, where cellulose microfibrils are meshed into a polysaccharide *matrix.*

Compared to starch, cellulose is also much more crystalline. Whereas starch undergoes a crystalline to amorphous transition when heated beyond 60-70 °C in water (as in cooking), cellulose requires a temperature of 320 °C and pressure of 25 MPa to become amorphous in water.

Several different crystalline structures of cellulose are known, corresponding to the location of hydrogen bonds between and within strands. Natural cellulose is cellulose I, with structures I_{α} and I_{β}. Cellulose produced by bacteria and algae is enriched in I_{α} while cellulose of higher plants consists mainly of I_{β}. Cellulose in regenerated cellulose fibers is cellulose II. The conversion of cellulose I to cellulose II is irreversible, suggesting that cellulose I is metastable and cellulose II is stable. With various chemical treatments it is possible to produce the structures cellulose III and cellulose IV. Many properties of cellulose depend on its chain length or degree of polymerization, the number of glucose units that make up one polymer molecule. Cellulose from wood pulp has typical chain lengths between 300 and 1700 units; cotton and other plant fibers as well as bacterial cellulose have chain lengths ranging from 800 to 10,000 units. Molecules with very small chain length resulting from the breakdown of cellulose are known as cellodextrins; in contrast to long-chain cellulose, cellodextrins are typically soluble in water and organic solvents.

Methyl cellulose (or methylcellulose) is a chemical compound derived from cellulose. It is a hydrophilic white powder in pure form and dissolves in cold (but not in hot) water, forming a clear viscous solution or gel. It is sold under a variety of trade names and is used as a thickener and emulsifier in various food and cosmetic products, and also as a treatment of constipation. Like cellulose, it is not digestible, not toxic, and not an allergen.

Methyl cellulose does not occur naturally and is synthetically produced by heating cellulose with caustic solution (e.g. a solution of sodium hydroxide) and treating it with methyl chloride. In the substitution reaction that follows, the hydroxyl residues (-OH functional groups) are replaced by methoxide (-OCH₃ groups).

Different kinds of methyl cellulose can be prepared depending on the number of hydroxyl groups substituted. Cellulose is a polymer consisting of numerous linked glucose molecules, each of which exposes three hydroxyl groups. The *Degree of Substitution* (DS) of a given form of methyl cellulose is defined as the average number of substituted hydroxyl groups per glucose. The theoretical maximum is thus a DS of 3.0, however more typical values are 1.3-2.6.Different methyl cellulose preparations can also differ in the average length of their polymer backbones.

Methyl cellulose has a lower critical solution temperature (LCST) between 40 °C and 50 °C. At temperatures below the LCST, it is readily soluble in water; above the LCST, it is not soluble, which has a paradoxical effect that heating a saturated solution of methyl cellulose will turn it solid, because methyl cellulose will precipitate out. The temperature at which this occurs depends on DS-value, with higher DS-values giving lower solubility and lower precipitation temperatures because the polar hydroxyl groups are masked. Preparing a solution of methyl cellulose with cold water is difficult: as the powder comes into contact with water, a gel layer forms around it, dramatically slowing the diffusion of water into the powder, hence the inside remains dry.

Carboxymethyl cellulose (CMC) or cellulose gum is a cellulose derivative with carboxymethyl groups (-CH₂-COOH) bound to some of the hydroxyl groups of the glucopyranose monomers that make up the cellulose backbone. It is often used as its sodium salt, sodium carboxymethyl cellulose.

CMC is used in food science as a viscosity modifier or thickener, and to stabilize emulsions in various products including ice cream. It is also a constituent of many non-food products, such as personal lubricants, toothpaste, laxatives, diet pills, water-based paints, detergents, textile sizing, and various paper products. It is used primarily because it has high viscosity, is nontoxic, and is generally considered to be hypoallergenic as the major source fiber is either softwood pulp or cotton linter. CMC is used extensively in gluten free and reduced fat food products. CMC is also used in pharmaceuticals as a thickening agent.

Sodium carboxymethyl cellulose, also known as croscarmellose sodium, is an internally cross-linked sodium carboxymethylcellulose. It is used as a superdisintegrant in pharmaceutical formulations.

The exemplary formula in Table 1 uses BLANOSE^{®} brand food-grade sodium carboxymethyl cellulose, commercially available from Ashland Chemical Co., Covington KY USA. Food-grade and pharmaceutical-grade sodium carboxymethyl cellulose is available from other vendors, e.g., Sigma-Aldrich Inc. and Spectrum Chemical, Inc. Other gelling agents may be used in addition to or in lieu of sodium carboxymethyl cellulose.

### Citric acid anhydrous

Citric acid is a commodity chemical, commercially available from a wide variety of suppliers. It is used mainly as an acidifier, as a flavoring, and as a chelating agent.

Citric acid is a weak organic acid with the formula C₆H₈O₇. It is a natural preservative/conservative and is also used to add an acidic or sour taste to foods and drinks. In biochemistry, the conjugate base of citric acid, citrate, is important as an intermediate in the citric acid cycle, which occurs in the metabolism of all aerobic organisms. It consists of 3 carboxyl (R-COOH) groups.

At room temperature, citric acid is a white crystalline powder. It can exist either in an anhydrous (water-free) form or as a monohydrate. The anhydrous form crystallizes from hot water, while the monohydrate forms when citric acid is crystallized from cold water. The monohydrate can be converted to the anhydrous form by heating above 78 °C.

The dominant use of citric acid is as a flavoring and preservative in food and beverages, especially soft drinks. The buffering properties of citrates are used to control pH in pharmaceuticals. I prefer that the citric acid used conform to the purity requirements for citric acid as a food additive are defined by the Food Chemicals Codex published by the United States Pharmacopoeia.

Without intending to be bound to any theoretical mechanism, I believe that the citric acid in my formulation functions as an emulsifying agent to keep the lipophilic lecithin oil from separating from the hydrophilic bromelain. Further, citric acid is an excellent chelating agent, binding metals. For example, it is used to remove limescale from boilers and evaporators. It can be used to soften water, which makes it useful in soaps and laundry detergents. By chelating the metals in hard water, it lets these cleaners produce foam and work better without need for water softening. Chelation activity is important in my formula because metal ion may interfere with the biological activity of bromelain.

### EPIKURON^{®} 135F lecithin oil

Lecithin has emulsification and lubricant properties, and is a surfactant. Commercial lecithin, as used by food manufacturers, is a mixture of phospholipids in oil. The lecithin can be obtained by water degumming the extracted oil of seeds. It is a mixture of various phospholipids, and the composition depends on the origin of the lecithin. A major source of lecithin is soybean oil. Other sources of lecithin (e.g., sunflower oil) may be used to avoid soy allergy concerns. The main phospholipids in lecithin from soya and sunflower are phosphatidyl choline, phosphatidyl inositol, phosphatidyl ethanolamine, and phosphatidic acid. They often are abbreviated to PC, PI, PE, and PA, respectively. Purified phospholipids are produced by companies commercially.

To modify the performance of lecithin to make it suitable for the product to which it is added, it may be hydrolyzed enzymatically. In hydrolysed lecithins, a portion of the phospholipids have one fatty acid removed by phospholipase. Such phospholipids are called lysophospholipids. The most commonly used phospholipase is phospholipase A2, which removes the fatty acid at the C2 position of glycerol. Lecithins may also be modified by a process called fractionation. During this process, lecithin is mixed with an alcohol, usually ethanol. Some phospholipids, such as phosphatidylcholine, have good solubility in ethanol, whereas most other phospholipids do not dissolve well in ethanol. The ethanol is separated from the lecithin sludge, after which the ethanol is removed by evaporation to obtain a phosphatidylcholine-enriched lecithin fraction.

Soybean-derived Lecithin dietary supplements are composed of 19-21% phosphatidylcholine, 8-20% Phosphatidylethanolamine, 20-21% Inositol phosphatides, 33-35% Soybean oil, 2-5% Sterols, 5% Carbohydrates/free, 1% Moisture, and 5-11% Other phosphatides. Lecithin is used for applications in human food, animal feed, pharmaceuticals, paints, and other industrial applications. In the pharmaceutical industry, lecithin acts as a wetting, stabilizing agent and a choline enrichment carrier, helps in emulsifications and encapsulation, and is a good dispersing agent. Lecithin is approved by the United States Food and Drug Administration for human consumption with the status "generally recognized as safe." Lecithin is also permitted by the European Union as a food additive (designated E322). The exemplary formula of Table 1 uses lecithin as an emulsifier. As an emulsifier, lecithin imparts several advantages. In animal feed, it enriches fat and protein content and improves pelletization. Research studies show soy-derived lecithin has significant effects on lowering serum cholesterol and triglycerides, while increasing HDL ("good cholesterol") levels in the blood of rats. It can be totally metabolized by humans, so is well tolerated by humans and non-toxic when ingested. (In contrast, certain other emulsifiers can only be excreted via the kidneys). Other emulsifiers, however, may be used in addition to or in lieu of lecithin.

### Ethylenedinitrilotetraacetic acid, disodium salt dihydrate ("EDTA")

Ethylenediaminetetraacetic acid, widely abbreviated as EDTA, is a chelating agent. It is colorless, water-soluble solid. Its conjugate base is ethylenediaminetetraacetate.

Its usefulness arises because of its role as a chelating agent, i.e. its ability to sequester metal ions such as Ca²⁺ and Fe³⁺. Chelating activity is advantageous for my formulation because metal ion (e.g., metal ion commonly present in tap water used in livestock farming) may interfere with or inhibit the activity of bromelain.

After being bound by EDTA, metal ions remain in solution but exhibit diminished reactivity. EDTA is produced as several salts, notably disodium EDTA and calcium disodium EDTA.

In industry, EDTA is mainly used to sequester metal ions in aqueous solution. In the textile industry, it prevents metal ion impurities from modifying colors of dyed products. EDTA inhibits the ability of metal ions, especially Mn²⁺, from catalyzing the disproportionation of hydrogen peroxide. In a similar manner, EDTA is added to some food as a preservative or stabilizer to prevent catalytic oxidative decoloration, which is catalyzed by metal ions. In soft drinks containing ascorbic acid and sodium benzoate, EDTA mitigates formation of benzene (a carcinogen).

EDTA is used to bind metal ions in the practice of chelation therapy, e.g., for treating mercury and lead poisoning. It is used in a similar manner to remove excess iron from the body. This therapy is used to treat the complication of repeated blood transfusions, as would be applied to treat thalassemia. The U.S. FDA approved the use of EDTA for lead poisoning on July 16, 1953, under the brand name of Versenate, which was licensed to the pharmaceutical company Riker. Some alternative medical practitioners believe EDTA acts as a powerful antioxidant to prevent free radicals from injuring blood vessel walls, therefore reducing atherosclerosis. The U.S. FDA has not approved it for the treatment of atherosclerosis.

Without intending to be bound by any hypothetical mode of action, EDTA may also serve in my formulation as a preservative, perhaps to enhance the preservative action of citric acid.

In addition to or in lieu of EDTA, one may use another chelating agent(s). For example, one could use a chelating ligand which binds metal ion but also has a higher biodegradability and a lower content of nitrogen than does EDTA.

For example, one may use Iminodisuccinic acid (IDS). Commercially used since 1998, iminodisuccinic (IDS) acid biodegrades about 80% after only 7 days. IDS binds to calcium exceptionally well and forms stable compounds with other heavy metal ions. In addition to having a lower toxicity after chelation than does EDTA, the production of IDS is environment-friendly. Additionally, IDS is degraded through the use of IDS-epimerase and C-N lyase found in *Agrobacterium tumefaciens* (BY6) which can be harvested on a large scale. Additionally, the reactions catalyzed by both enzymes does not require any cofactors and can thus be applied directly.

Similarly, one may use Polyaspartic acid. Polyaspartic acid, commercially available as BAYPURE^{™} DS 100, is produced in an environmentally friendly manner. Polyaspartic acid, like Iminodisuccinic acid binds to calcium and other heavy metal ions. It has a higher value of 7.2 meq/g than does EDTA, which only has 6.0 meq/g. While it has a higher theoretical capacity, in practical applications it exhibits low efficiency in lower ion concentration solutions. DS has many practical applications including corrosion inhibitors, waste water additives, and agricultural polymers. A BAYPURE^{™} DS 100 based laundry detergent was the first laundry detergent in the world to achieve the EU flower ecolable.

Similarly, one may use Ethylenediamine-N,N'-disuccinic acid (EDDS). As a structural isomer of EDTA, ethylenediamine-N,N'-disuccinic acid can exist three isomers: (S,S), (R,S)/(S,R) and (R,R), but only the S,S-isomer is readily biodegradable. EDDS exhibits a surprisingly high rate biodegradation at 83% in 20 days. Biodegradation rates also varies the different metal ions chelated. For example, the complexes of lead and zinc with EDDS have relatively the same stability but the lead complex is biodegrades more efficiently than the zinc complex. As of 2002, EDDS has been commercially prominent in Europe on a large scale with an estimated demand rate increase of about 15% each year.

Similarly, one may use Methylglycinediacetic acid (MGDA). Commercially available from BASF GmbH, methylglycinediacetic acid (MGDA) is produced from glycine. MGDA has a high rate of biodegradation >68%, but unlike many other chelating agents can degrade without the assistance of adapted bacteria. Additionally, unlike EDDS or IDS, MGDA can withstand higher temperatures while maintaining a high stability as well as the entire pH range. As a result, the chelating strength of MGDA is stronger than many commercial chelating agents.

Similarly, one may use L-glutamic acid N,N-diacetic acid, tetra sodium salt (GLDA). Such aminopolycarboxylate-based chelates are used to control metal ions in water-based systems.

### EXAMPLES

I tested my formulation on swine grown in three different types of environments and three different climates, under actual commercial farming conditions, assessing two different age groups of pigs - suckers (unweaned) and weaners.
- Trial 1 (Spain) - My formulation reduced the incidence, severity and duration of post-weaning scour in piglets. My formulation also reduced the requirement for antibiotics and improved feed conversion ratios (FCR).
- Trial 2 (France) - My formulation improved average daily weight gain, and improved food conversion ratios when compared to antibiotics in feed.
- Trial 3 (Philippines) - My formulation reduced piglet deaths in sucker piglets compared with antibiotics.
- Trial 4 (Australia) - My formulation reduced piglet deaths in sucker piglets compared with antibiotics.

### EXAMPLE 1 - Spain (just weaned piglets).

Aim: The objective of this study was to compare whether a single oral dose of my formulation (4 ml) at weaning could reduce scour in a commercial piggery with a history of E. coli.

Study Outline: This study was a blinded, randomised field trial comparing two parallel groups of piglets. I used two Test Groups (n = 72 per group): 1. My formulation, and 2. No treatment. On the day of weaning (day 0), piglets in each litter were randomly assigned to two different treatment groups, weighed and then given a unique identification (ID) number. A single dose of my formulation was then administered to one group of piglets which were then transported to the weaning pens. The other group of piglets were left untreated, but handled in an identical manner.

Clinical Parameters: Piglets were monitored daily for scour and signs of any other disease. Once piglets showed signs of scour, their ID numbers, fecal consistency and the general condition of piglets were recorded using a scoring system (Table 2).

**Table 2. Scoring fecal consistency and piglet general condition.**

| Fecal consistency | General condition |
|---|---|
| 0: normal | 0: normal |
| 1: pasty or partially formed (mild) | 1: mildly depressed |
| 2: loose, semi liquid (moderate) | 2: severely depressed. |
| 3: profuse and watery (severe) | |

Classification of the animals as healthy, unwell or moribund were based on the total clinical score (Table 3). The total clinical score is the sum of the fecal consistency score plus the general condition score for each pig. This score gives an overall indication of piglet health. Some piglets may have scour, but still appear healthy, whereas other piglets may have mild scour, but be moribund. My formulation significantly reduced the clinical score of piglets, and therefore improved their overall health compared to untreated pigs.

**Table 3. Classification of the piglets by their total clinical score.**

| Total clinical score | Classification |
|---|---|
| 0 | Healthy |
| 1 | Healthy |
| 2 | Unwell |
| 3 | Moribund and requires individual animal treatment. |
| 4+ | Moribund and requires removal and/or euthanasia |

Antibiotic treatments: All antibiotic treatments administered during the study were recorded (animal ID, date, product, dose and route of administration).

Piglet Weight: Piglets were individually weighed on day 0 (at weaning), day 7 and day 14. The average daily weight gain (ADG) was calculated.

Feed Intake: The feed intake per pen was also assessed to determine feed conversion ratios (FCR). The FCR is equal to the feed intake divided by the weight of the pig.

Data Analysis: To evaluate the incidence of scour, clinical score or morbidity (general condition + fecal consistency), the treatment rate and mortality rate, the statistical procedures used was a Linear Mixed Model with poisson and binomial errors. Room/Sex were as random effects and Treatment as a fixed effect. Analyses were conducted with GenStat for Windows. (2007). 10th Edn. VSN International Ltd., Hemel Hempstead, UK. For body weight and average daily weight gain the statistical procedures used were Linear Mixed Models with normal errors. The Type 1 error was ≤ 0.05.

### Results:

### Clinical Parameters:

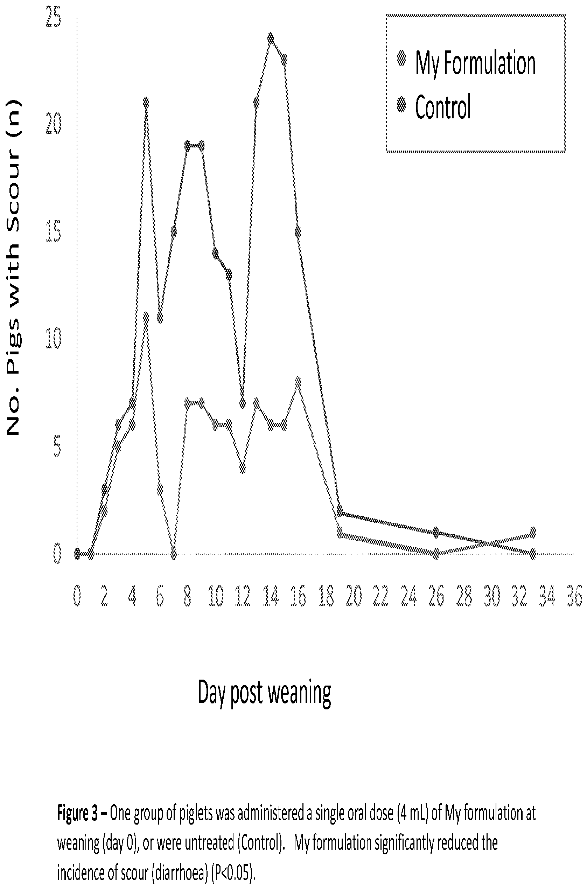

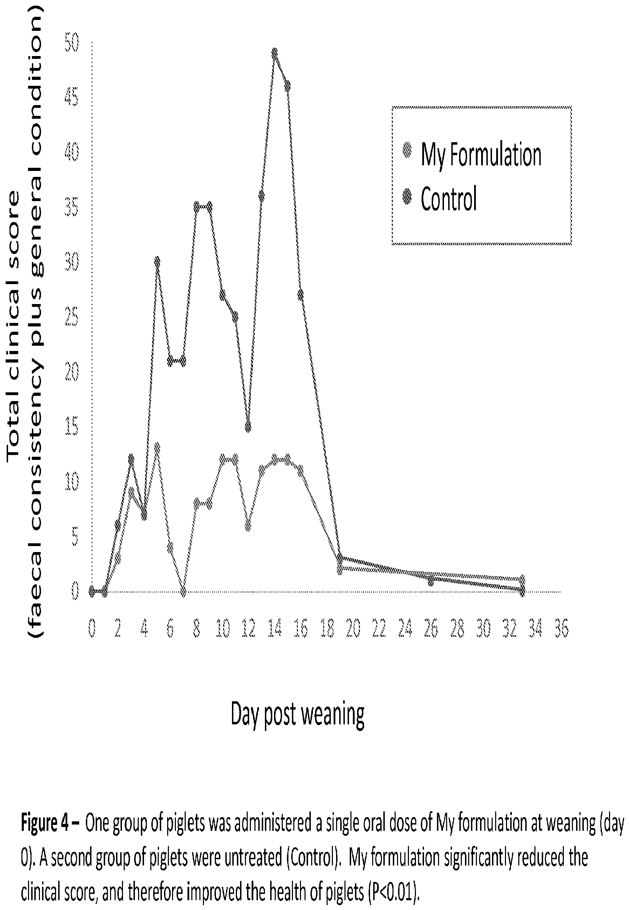

- A single dose of my formulation administered at weaning significantly reduced the incidence of scour (from day 0 to day 19 post weaning) by 40% when compared to untreated pigs (Figure 3) (p<0.05). This single dose protected piglets for 19 days, indicating a long duration of effect. The cause of scour on this farm was E. coli.
- Over the duration of the study, the total diarrhea score (sum of all diarrhea scores) in the group of piglets treated with my formulation was 98, compared with 253 in the untreated group.
- My formulation significantly improved the overall health in piglets or they had a reduced clinical score, therefore less severe disease, when compared with untreated piglets (Figure 4) (p<0.05).
- My formulation significantly reduced the number of sick pigs by 58% (n = 16) when compared to untreated pigs (n = 38) (p<0.05).
- My formulation reduced the requirement for the number of antibiotic treatments by 55% (15 treatments) versus controls (33 treatments).
- There were equal numbers of deaths (all causes) in the group treated with my formulation (4) and in the untreated group (3).

### Pig Performance:

- The average daily weight gain in piglets treated with my formulation in the two weeks post weaning was 22% higher than in untreated piglets (50 ± 7.1 g vs 39±7.0 g, respectively). At 42 days post-weaning, my formulation treated piglets were 0.2 kg (1.6%) heavier than control piglets, but this increase was not significant.
- In the first 2 weeks after weaning, piglets treated with my formulation had a significantly better feed conversion ratio of 8.4% than untreated pigs (2.84 ± 1.22 vs 3.1 ± 1.20, respectively). Overall (day 0 to 42) my formulation had a 2.7% improvement in feed conversion ratios (1.46 ± 0.06 versus 1.50 ± 0.06).

Although the overall improved FCR of my formulation compared to the untreated group is modest (0.04 or 2.7%), it should be noted that every 0.01 improvement in FCR will reduce feed cost by $0.28 to $0.30 per pig (based on 2008 figures - cost of feed has gone up significantly, so current benefits will be greater). It has also been calculated that a 0.1 % improvement in grower FCR can improve the profitability of a 200-sow unit by approximately $6,000 per annum. A 5% improvement in FCR has a potential value of $28 million to the Australian pork Industry (http://www.australianpork.com.au).

The improved performance in FCR is important, as the cost of feed is the major cost to pork production, usually accounting for over 60 to 80 percent of all production expenses. Every improvement in FCR will reduce feed costs and improve profitability.

### Conclusion:

A single dose of my formulation administered at weaning reduces the incidence, duration and severity of scour. My formulation also reduces the requirement for antibiotic treatments, and improves growth in piglets. Therefore, my formulation improves piglet health and performance, and therefore farm productivity.

### EXAMPLE 2 - France (weaned piglets).

Background: Antibiotics as a feed additive to promote growth is now banned in Europe, however, addition of prescribed antibiotics to feed under veterinary supervision is allowed for the prophylaxis and treatment of acute conditions, such as scour.

Aim: The objective of this study was to compare the feed conversion ratios of piglets administered a single dose of my formulation with in feed colistin, an antibiotic.

Study Outline: This study was a blinded, controlled, randomised field trial comparing three parallel groups of piglets, (n=89 per group): 1. Colistin; 2. My formulation; and 3. No treatment. In this study, whole litters are randomised to receive different treatments, so litters are treated on a whole litter basis.

Weaning on this farm occurs in 2 stages. In stage 1, piglets are weaned by removing the sow (at day -5). Then on day 0, piglets are moved to their weaning pen.

At day -5, Group 1 piglets were administered colistin (9 kg antibiotic premix per Ton of feed) in pre-starter feed for 14 days (day -5 to day 9). The other two groups were administered pre-starter feed alone. On day 0 (5 days post weaning), when piglets are moved to their weaner pens, Group 2 piglets received a single dose of my formulation. Group 3 piglets were untreated.

Analysis: As per Study 1.

### Results:

### Pig Performance

- My formulation treated piglets had a significantly higher average daily weight gain during all phases of the study compared to piglets receiving colistin (P<0.05, Table 4). Piglets treated with colistin in their feed had the lowest weight gain of all groups, including less weight gain than untreated pigs (P<0.05).

**Table 4 - Average Daily Weight gain (g) of all groups.**

| | My formulation | Colistin | Untreated |
|---|---|---|---|
| Pre-starter (d-5 to d9) | 252 ± 74 | 167 ± 67 | 236 ± 63 |
| Starter | 537 ± 105 | 505 ± 110 | 542 ± 110 |
| Post weaning | 429 ± 81 | 377 ± 82 | 426 ± 75 |
| Post weaning and fattening (overall) | 692 ± 65 | 664 ± 67 | 678 ± 56 |

• Overall (at 150 days of age) the average total weight gain of piglets administered my formulation was 2% (or 1.71 kg) higher than control pigs, and 3.9% (or 3.25 kg) higher than piglets administered colistin in feed (P<0.05).
• The feed intake was determined for the pre-starter phase only (d-5 to d9). My formulation treated piglets had a higher feed intake over the weaning period compared to piglets receiving colistin in feed (Table 5).

**Table 5. Feed intake (g) during the pre-starter phase**

| | My formulation | Colistin | Control |
|---|---|---|---|
| Number of pens | 6 | 6 | 6 |
| Min - Max | 408 - 499 | 358 - 479 | 420 - 529 |
| Arithmetic Mean | 450 | 417 | 460 |

• The feed conversion ratio (FCR) was determined for the pre-starter phase only (d-5 to d9). My formulation alone treated piglets had the best FCR (1.82) (P<0.009) than all the other groups (Table 5). Piglets treated with my formulation had a 33% improvement over piglets treated with colistin (2.71). Despite piglets treated with my formulation having a lower feed intake than untreated pigs (Table 5), they still had a 7% improvement in FCR over untreated pigs (1.96) (Table 6). These performance results show that my formulation has a significant advantage over colistin in feed.

**Table 6. Feed Conversion Ratio during the pre-starter phase per Group**

| | My formulation | Colistin | Control |
|---|---|---|---|
| Number of pens | 6 | 6 | 6 |
| Median | 1.77 | 2.59 | 2.01 |
| Min - Max | 1.59 - 2.10 | 1.75 - 3.42 | 1.63 - 2.11 |
| Arithmetic Mean | 1.82 | 2.71 | 1.96 |

The negative effect of colistin on FCR may be because of its adverse effect on the gut flora, and thus a negative effect on pig gut health and nutrition.

Conclusion: My formulation improved weight gains of piglets and improved food conversion ratios.

### EXAMPLE 3 - Philippines (unweaned piglets or sucker piglets).

Background: This farm in the Philippines has a high death rate due to scour.

Aim: The objective of this study was to compare the efficacy of my formulation with antibiotics in unweaned piglets.

Study Outline: This study was a randomised field trial comparing two groups of piglets. Since the mortality rate on this farm is very high, there is no negative control (non-medicated group). Test Groups (n = 38 per group) were 1. My formulation (2 doses), and 2. Antibiotics (orally administered every 3 days). Piglets received my formulation at 3 days of age. A follow up dose was given at 6 days of age.

### Results:

### Clinical Parameters

- There were few deaths due to scour in this study. But there were significantly lower deaths of 5% (from all causes) in the group treated with my formulation. The death rate was 21% in piglets receiving antibiotics (P<0.05).

### Performance Parameters

- At weaning, piglets receiving my formulation weighed 0.1 kg more than piglets treated with antibiotics. Since there were no negative controls in this study, it is unknown whether my formulation had increased weight over untreated pigs, as observed in other field trials.

### EXAMPLE 4 - Australia (unweaned piglets or sucker piglets).

Aim: The objective of this study was to investigate the efficacy of my formulation (2 mL) in reducing piglet mortality and morbidity on an Australian farm with a history of pre-weaning (sucker) scour.

Study Outline: The study was conducted on a commercial piggery located in Northern Victoria, Australia. This farm has a history of problems with pre-weaning scour usually occurring at 3-4 days following birth. Faecal samples obtained from the farm in the month prior to the study indicated that the scour was due to a combined infection of *E. coli* (K99, STa toxin genes) and Rotavirus. Current approaches such as vaccines as well as antibiotics had failed to adequately control the problem.

This study was a blinded, placebo controlled, randomised field trial comparing two parallel groups of piglets. The farmer and farm workers were blinded to the treatments.

There were 21 litters (233 piglets) administered my formulation (2 mL) at 2 days of age (Group 1), while 23 litters (229 piglets) received a placebo (Group 2).

Each group contained equal gilt litters (or first time mothers).

The usual management routines of the farm were allowed to continue, including usual medications such as sow vaccinations, antibiotics, and coccidiostats, as well as cross fostering of piglets, where small piglets or those of ill health may be moved to another sow.

The trial was conducted by independent veterinarians and investigators from the Pig Specialist Centre, Victorian Department of Economic Development, Jobs, Transport and Resources. The appropriate statistical analysis was determined and applied by an independent biometrician.

The trial investigated the incidence of death and scour, morbidity (or piglet clinical condition), as well as weight gains, and average daily weight gain (ADG) from 2 to 21 days of age. Piglets were monitored daily for scour and signs of any other disease. Once piglets showed signs of scour, their ID numbers, faecal consistency and the general condition of piglets were recorded using the scoring system described in Table 2.

Any piglet found to be severely depressed was euthanized on humane grounds. Piglets euthanized or found dead during the trial were necropsied within 12 hours of death.

### Data Analysis:

There were 21 - 23 replications of the two treatments randomly positioned in a shed.

A litter of piglets in a pen is the experimental unit.

The appropriate statistical analysis was determined and applied by an independent biometrician.

Average maximum scour scores and morbidity scores per pen were analysed in a one way Analysis of Variance (ANOVA) after logₑ + 0.05 transformed to stabilize variances. ADG for all piglets was unsuitable for ANOVA because residuals were neither normally distributed or homogeneous over the range of fitted values; so we used the non-parametric (distribution free) Kruskal-Wallis test.

Mortalities and the incidences of scouring and morbidity were analysed using Exact Binary Regression, suitable for small cell sizes. Two tailed tests of significance were used.

ANOVAs and the Kruskal Wallis Test were performed using R version 2.7.2 (2008). The R Foundation for Statistical Computing. Exact Binary Regression was performed with StatExact (Cytel Statistical Software, Cytel Software Corporation, MA, USA).

### Results:

### Clinical Parameters

The study was designed as a prophylactic study, where my formulation was to be administered to piglets prior to the expected onset of scour. However, prior to product administration, scour was evident in 59 of 462 piglets (12.8%). Despite the earlier than expected onset of scour, all piglets were included in the study, and there were no exclusions.

Table 7 shows the number of pre-weaning mortalities in both groups due to all causes. My formulation significantly reduced piglet mortality by 47.8% (p < 0.02). Of the piglets treated with my formulation, 19 of 233 (8.2%) piglets died compared with 36 of 229 (15.7%) piglets which died in the control group.

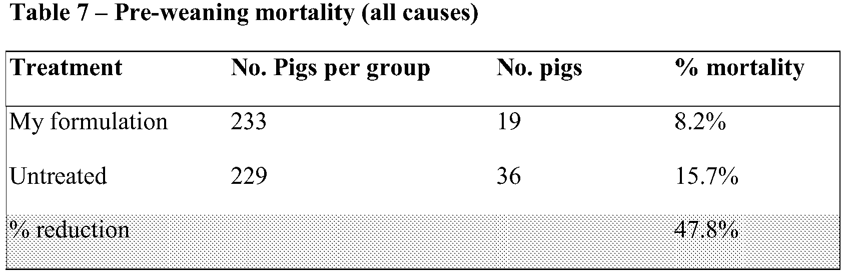

The primary cause of death of piglets in this study (81.8%) was diagnosed as scour and ill thrift based on post-mortem findings.

### Etiology

Samples were collected from 24 piglets that were less than 7 days of age. No predominant pathogen was identified in this study. Twenty three faecal samples were subject to culture (aerobic and anaerobic) to isolate possible bacterial causes of diarrhoea (*E. coli* and Clostridia). One piglet treated with my formulation tested positive for non-haemolytic *E. coli* (STa positive), and two haemolytic *E coli* isolates (K88) were obtained from two piglets from the Control group. One of these Control piglets had a co-infection with non-haemolytic *E. coli* (K88). No Clostridia *spp.* were isolated. Nine of 21 (42.8%) faecal samples tested as positive or weakly positive by Rotavirus ELISA (IDEXX Rota-Corona-K99, IDEXX Montpellier SAS, France). However, none of 7 samples were positive on the rotavirus RTPCR, nor had intestinal lesions indicative of rotavirus infections.

A high proportion 26/53 (49%) of piglets autopsied had empty stomachs suggesting that the diarrhoea/ill-thrift present on this farm could have been attributed to inadequate colostrum and milk intake in piglets, leading to sub-optimal nutritional intake in piglets and poor lactogenic protection.

### Morbidity

My formulation also reduced severe morbidity, or life threatening disease (Score 4). Of the piglets that had life threatening disease, or were considered moribund, 36 of 38 control piglets died, compared with 19 of 28 piglets that were moribund in group treated with my formulation.

### Performance Parameters

Table 8 shows the mean and range of weight gains (from day of treatment, Day 2 to Day 21) and the average daily weight gain (ADG) for both groups.

**Table 8 - Weight gains and ADG from Day 2 to weaning at 21 days.**

| Treatment | No. Pens | Litter size (weaned) | Average Weight Gain (g) (min - max) | Average ADG (g/day) (min - max) |
|---|---|---|---|---|
| My Formulation | 21 | 10.19 | 4,188 (2,512 to 5,279) | 199 (120 to 251) |
| Control | 23 | 8.48 | 3,964 (939 to 5,205) | 189 (45 to 248) |
| % increase | | | 5.7% | 5.6% |

My formulation increased the weight gain and average daily weight gain by 5.7% (or 224 g per piglet) and 5.6%. But these gains were not statistically significant (p=0.49) at the litter level (but was significant at the individual piglet level, p<0.04).

### Discussion and Conclusion:

The results of this study suggest that my formulation can be used reduce pre-weaning mortality on farms that have a problem with diarrhea/ill-thrift of non-specific etiology.

Ill-thrift and failure-to-thrive is a major cause of death among piglets in the first week of life. Piglets that fail to ingest colostrum in the first 24 hours after birth are at risk of early death as a result of crushing by the sow or exposure due to inadequate energy intake. Unlike human babies, no antibodies are transferred to piglets via the placenta from the sow. So without maternal antibodies, the piglet is highly susceptible to infection. If they survive the first few days, but continue to have inadequate milk intake, piglets are more likely to succumb to infectious disease due to low lactogenic immunity (from milk antibodies and immune factors in the sow's milk) compared with their more robust littermates. Those that fail to sustain adequate milk intake, either through poor milk production by the sow or low consumption by the piglet are again more likely to succumb to an early death due to an inability to compete with littermates or to infectious disease later in life.

In this study, my formulation halved the pre-weaning mortality rate among sucker piglets on a commercial farm. It would appear that its mode of action was through improving the vigour and therefore the survivability of moribund piglets in the first week of life. This is demonstrated by the lower proportion (45.2%) of piglets treated with my formulation that were classified as clinically moribund that died or had to be euthanized, compared with 76.6% in the Control group in the same clinical category.

Piglets treated with my formulation grew 5.7% faster than piglets in the control group. Although this weight gain difference was not significant, this equates to approximately a 225 g difference in live-weight at weaning. Weaning weight is positively associated with subsequent growth and survival of piglets.

### Summary

Given my disclosure here, one may readily make certain variants and alternatives. For example, the formulation of Table 1 provides a dosage suitable for prophylactic treatment of a suckling piglet to prevent scour. Use to treat (rather than prevent) scour may require a different dose; the artisan would readily be able to derive the appropriate dose. Similarly, use to treat a mature adult pig, or a human may require a larger dose; the artisan would readily be able to derive the appropriate dose.

One may provide my formulation as an oral drench, for example as a granulated powder requiring reconstitution with water. To prevent post-weaning scour, my formulation may be given as a once only oral dose 4 ml (0.24 g) on the day of weaning (1-2 days before the expected on set of scour). To prevent pre-weaning scour, a 2 ml (0.12 g) single oral dose can be administered at 2-5 days of age, depending on a particular farm's problem period. A repeat dose may be required 3-7 days later. As a treatment, my formulation may be administered (either 2 ml or 4 ml) immediately when symptoms of disease occur.

One may provide my formulation as a feed additive, for example prepared as a granulated powder that can added to pig feed. To ensure thorough dispersion of the product it should first be mixed with a suitable quantity of feed ingredients before incorporation in the final mix. My formulation may be fed as a pre-mix only, or the pre-mix incorporated in the final mix. The recommended dose level is 40 mg my formulation /kg bodyweight fed daily for 14 consecutive days.

One may also deliver my formulation in water via drinking systems. Alternatively, one may use bromelain to make an equivalent oral drench, formulated with excipients and requiring reconstitution in liquid. To prevent post-weaning scour, this may be given as a once only oral dose (125 mg) on the day of weaning (1-2 days before the expected on set of scour). To prevent pre-weaning scour, a 62.5 mg single oral dose may be administered at 2-5 days of age, depending on a particular farm's problem period. A repeat dose may be required 3-7 days later. As a treatment, it may be administered (either 62.5 mg or 125 mg) immediately when symptoms of disease occur.

Alternatively, one may use bromelain to make an equivalent feed additive, for example as a powder that can added to pig feed. To ensure thorough dispersion of the product it should first be mixed with a suitable quantity of feed ingredients before incorporation in the final mix. It may be fed as a pre-mix only, or the pre-mix incorporated in the final mix. The recommended dose level is 20 mg bromelain/kg bodyweight fed daily for 14 consecutive days.

Alternatively, my formulation may be provided as tablet and capsules, and other appropriate dose forms for humans.

The skilled artisan may adjust my formulation for different indications. For example, it may be used for the prevention and treatment of scour in production animals (cattle, swine etc.) and diarrhea in humans. It may also be used for improved gut health by reducing inflammation. Alternatively, it may be formulated to promote increased feed intake in production animals, thus promoting weight gains and feed conversion efficiency. It may be used to reduce the requirement for antibiotics in animal feed, and for acute administration to humans. It may also be used to ameliorate Inflammatory Bowel Disease in humans.

## Claims

1. An oral veterinary formulation comprising an anti-diarrhea effective amount of bromelain, wherein the formulation comprises an emulsifier in an amount effective to improve the solubility of said bromelain in water,
wherein the emulsifier comprises lecithin, and
wherein the formulation further comprises citric acid.

2. The oral veterinary formulation of claim 1, further comprising sodium carboxymethyl cellulose and EDTA, wherein the formulation is an aqueous suspension.

3. The oral veterinary formulation of claim 1, wherein the formulation comprises 53.1 - 74.7 wt% bromelain, 22 - 36 wt% sodium carboxymethyl cellulose, 4 - 12.7 wt% citric acid anhydrous, 3 - 7.9 wt% lecithin oil, and 0.7 - 1.6 wt% EDTA, and wherein the formulation is a free-flowing powder.

4. The oral veterinary formulation of claim 1 or 3, wherein the formulation comprises 62.5 - 88.4 mg bromelain, 25.9 - 42.4 mg sodium carboxymethyl cellulose, 4.7 - 14.96 mg citric acid anhydrous, 3.5 - 9.3 mg lecithin oil, and 0.8 - 1.9 mg EDTA per unit dose, and wherein the formulation is a free-flowing powder.

5. An oral veterinary formulation comprising the oral veterinary formulation according to claim 3 or 4 suspended in water.

6. The oral veterinary formulation according to any one of claims 3-5 for use in a method for reducing the likelihood of scour in piglets, the method comprising administering to the piglet a single dose of a therapeutically effective amount of the oral veterinary formulation.

7. The oral veterinary formulation according to any one of claims 3-5 for use in a method for the treatment of scour in a population of piglets, the method comprising administering to each piglet in the piglet population, a single dose of a therapeutically effective amount of the oral veterinary formulation.

8. An oral veterinary formulation for use in a method of treating scour in a piglet, wherein the oral veterinary formulation comprises an anti-diarrhea effective amount of bromelain and an emulsifier in an amount effective to improve the solubility of said bromelain in water, wherein the emulsifier comprises lecithin, and wherein the formulation further comprises citric acid, and wherein the piglet is unweaned or weaning.

9. The oral veterinary formulation for the use according to claim 8, further comprising sodium carboxymethyl cellulose and EDTA, wherein the formulation is an aqueous suspension.

10. The oral veterinary formulation for the use according to claim 8, wherein the formulation comprises 53.1 - 74.7 wt% bromelain, 22 - 36 wt% sodium carboxymethyl cellulose, 4 - 12.7 wt% citric acid anhydrous, 3 - 7.9 wt% lecithin oil, and 0.7 - 1.6 wt% EDTA, and wherein the formulation is a free-flowing powder.

11. The oral veterinary formulation for the use according of claim 8, or 10, wherein the formulation comprises 62.5 - 88.4 mg bromelain, 25.9 - 42.4 mg sodium carboxymethyl cellulose, 4.7 - 14.96 mg citric acid anhydrous, 3.5 - 9.3 mg lecithin oil, and 0.8 - 1.9 mg EDTA per unit dose, and wherein the formulation is a free-flowing powder.

12. An oral veterinary formulation for use in a method of treating scour in a piglet, wherein the oral veterinary formulation comprising an anti-diarrhea effective amount of bromelain and an emulsifier in an amount effective to improve the solubility of said bromelain in water, and wherein the piglet is unweaned or weaning, and wherein the formulation comprises the oral veterinary formulation according to claim 11 or 12 suspended in water.

13. The oral veterinary formulation for the use according to any one of claims 10-12, wherein the method comprising administering to the piglet a single dose of a therapeutically effective amount of the oral veterinary formulation.

## Patentansprüche

1. Orale tiermedizinische Formulierung, eine gegen Durchfall wirksame Menge Bromelain umfassend, wobei die Formulierung einen Emulgator in einer Menge umfasst, die wirksam ist, um die Löslichkeit des Bromelains in Wasser zu verbessern,
wobei der Emulgator Lecithin umfasst, und
wobei die Formulierung ferner Zitronensäure umfasst.

2. Orale tiermedizinische Formulierung nach Anspruch 1, ferner Natriumcarboxymethylcellulose und EDTA umfassend, wobei die Formulierung eine wässrige Suspension ist.

3. Orale tiermedizinische Formulierung nach Anspruch 1, wobei die Formulierung 53,1 - 74,7 Gew.-% Bromelain, 22 - 36 Gew.-% Natriumcarboxymethylcellulose, 4 - 12,7 Gew.-% wasserfreie Zitronensäure, 3 - 7,9 Gew.-% Lecithinöl und 0,7 - 1,6 Gew.-% EDTA umfasst und wobei die Formulierung ein rieselfähiges Pulver ist.

4. Orale tiermedizinische Formulierung nach Anspruch 1 oder 3, wobei die Formulierung 62,5 - 88,4 mg Bromelain, 25,9 - 42,4 mg Natriumcarboxymethylcellulose, 4,7 - 14,96 mg wasserfreie Citronensäure, 3,5 - 9,3 mg Lecithinöl und 0,8 - 1,9 mg EDTA pro Einheitsdosis umfasst und wobei die Formulierung ein rieselfähiges Pulver ist.

5. Orale tiermedizinische Formulierung, umfassend die orale tiermedizinische Formulierung nach Anspruch 3 oder 4 suspendiert in Wasser.

6. Orale tiermedizinische Formulierung nach einem der Ansprüche 3-5 zur Verwendung in einem Verfahren zum Verringern der Wahrscheinlichkeit von Durchfall bei Ferkeln, wobei das Verfahren Verabreichen einer Einzeldosis einer therapeutisch wirksamen Menge der oralen tiermedizinischen Formulierung an das Ferkel umfasst.

7. Orale tiermedizinische Formulierung nach einem der Ansprüche 3-5 zur Verwendung in einem Verfahren zur Behandlung von Durchfall in einer Ferkelpopulation, wobei das Verfahren Verabreichen einer Einzeldosis einer therapeutisch wirksamen Menge der oralen tiermedizinischen Formulierung an jedes Ferkel in der Ferkelpopulation umfasst.

8. Orale tiermedizinische Formulierung zur Verwendung in einem Verfahren zum Behandeln von Durchfall bei einem Ferkel, wobei die orale tiermedizinische Formulierung eine gegen Durchfall wirksame Menge Bromelain und einen Emulgator in einer Menge umfasst, die wirksam ist, um die Löslichkeit des Bromelains in Wasser zu verbessern, wobei der Emulgator Lecithin umfasst und wobei die Formulierung ferner Zitronensäure umfasst und wobei das Ferkel nicht abgesetzt oder abgesetzt ist.

9. Orale tiermedizinische Formulierung zur Verwendung nach Anspruch 8, ferner Natriumcarboxymethylcellulose und EDTA umfassend, wobei die Formulierung eine wässrige Suspension ist.

10. Orale tiermedizinische Formulierung zur Verwendung nach Anspruch 8, wobei die Formulierung 53,1 - 74,7 Gew.-% Bromelain, 22 - 36 Gew.-% Natriumcarboxymethylcellulose, 4 - 12,7 Gew.-% wasserfreie Zitronensäure, 3 - 7,9 Gew.-% Lecithinöl und 0,7 - 1,6 Gew.-% EDTA umfasst und wobei die Formulierung ein rieselfähiges Pulver ist.

11. Orale tiermedizinische Formulierung zur Verwendung nach Anspruch 8 oder 10, wobei die Formulierung 62,5 - 88,4 mg Bromelain, 25,9 - 42,4 mg Natriumcarboxymethylcellulose, 4,7 - 14,96 mg wasserfreie Citronensäure, 3,5 - 9,3 mg Lecithinöl und 0,8 - 1,9 mg EDTA pro Einheitsdosis umfasst und wobei die Formulierung ein rieselfähiges Pulver ist.

12. Orale tiermedizinische Formulierung zur Verwendung in einem Verfahren zum Behandeln von Durchfall bei einem Ferkel, wobei die orale tiermedizinische Formulierung eine gegen Durchfall wirksame Menge Bromelain und einen Emulgator in einer Menge umfasst, die wirksam ist, um die Löslichkeit des Bromelains in Wasser zu verbessern, und wobei das Ferkel nicht abgesetzt oder abgesetzt ist und wobei die Formulierung die orale tiermedizinische Formulierung nach Anspruch 11 oder 12 suspendiert in Wasser umfasst.

13. Orale tiermedizinische Formulierung zur Verwendung nach einem der Ansprüche 10-12, wobei das Verfahren Verabreichen einer Einzeldosis einer therapeutisch wirksamen Menge der oralen tiermedizinischen Formulierung an das Ferkel umfasst.

## Revendications

1. Formulation vétérinaire orale comprenant une quantité de bromélaïne efficace contre la diarrhée, dans laquelle la formulation comprend un émulsifiant en une quantité efficace pour améliorer la solubilité de ladite bromélaïne dans l'eau, dans laquelle l'émulsifiant comprend de la lécithine, et
dans laquelle la formulation comprend en outre de l'acide citrique.

2. Formulation vétérinaire orale selon la revendication 1, comprenant en outre de la carboxyméthylcellulose sodique et de l'EDTA, dans laquelle la formulation est une suspension aqueuse.

3. Formulation vétérinaire orale selon la revendication 1, dans laquelle la formulation comprend 53,1 à 74,7 % en poids de bromélaïne, 22 à 36 % en poids de carboxyméthylcellulose sodique, 4 à 12,7 % en poids d'acide citrique anhydre, 3 à 7,9 % en poids d'huile de lécithine et 0,7 à 1,6 % en poids d'EDTA, et dans laquelle la formulation est une poudre fluide.

4. Formulation vétérinaire orale selon la revendication 1 ou 3, dans laquelle la formulation comprend 62,5 à 88,4 mg de bromélaïne, 25,9 à 42,4 mg de carboxyméthylcellulose sodique, 4,7 à 14,96 mg d'acide citrique anhydre, 3,5 à 9,3 mg d'huile de lécithine et 0,8 à 1,9 mg d'EDTA par dose unitaire, et dans laquelle la formulation est une poudre fluide.

5. Formulation vétérinaire orale comprenant la formulation vétérinaire orale selon la revendication 3 ou 4 en suspension dans l'eau.

6. Formulation vétérinaire orale selon l'une quelconque des revendications 3 à 5 pour une utilisation dans un procédé de réduction du risque de diarrhée chez des porcelets, le procédé comprenant l'administration au porcelet d'une dose unique d'une quantité thérapeutiquement efficace de la formulation vétérinaire orale.

7. Formulation vétérinaire orale selon l'une quelconque des revendications 3 à 5 pour une utilisation dans un procédé de traitement de la diarrhée chez une population de porcelets, le procédé comprenant l'administration à chaque porcelet de la population de porcelets, d'une dose unique d'une quantité thérapeutiquement efficace de la formulation vétérinaire orale.

8. Formulation vétérinaire orale pour une utilisation dans un procédé de traitement de la diarrhée chez un porcelet, dans laquelle la formulation vétérinaire orale comprend une quantité de bromélaïne efficace contre la diarrhée et un émulsifiant en une quantité efficace pour améliorer la solubilité de ladite bromélaïne dans l'eau, dans laquelle l'émulsifiant comprend de la lécithine, et dans laquelle la formulation comprend en outre de l'acide citrique, et dans laquelle le porcelet est non sevré ou en cours de sevrage.

9. Formulation vétérinaire orale pour l'utilisation selon la revendication 8, comprenant en outre de la carboxyméthylcellulose sodique et de l'EDTA, dans laquelle la formulation est une suspension aqueuse.

10. Formulation vétérinaire orale pour l'utilisation selon la revendication 8, dans laquelle la formulation comprend 53,1 à 74,7 % en poids de bromélaïne, 22 à 36 % en poids de carboxyméthylcellulose sodique, 4 à 12,7 % en poids d'acide citrique anhydre, 3 à 7,9 % en poids d'huile de lécithine, et 0,7 à 1,6 % en poids d'EDTA, et dans laquelle la formulation est une poudre fluide.

11. Formulation vétérinaire orale pour l'utilisation selon la revendication 8 ou 10, dans laquelle la formulation comprend 62,5 à 88,4 mg de bromélaïne, 25,9 à 42,4 mg de carboxyméthylcellulose sodique, 4,7 à 14,96 mg d'acide citrique anhydre, 3,5 à 9,3 mg d'huile de lécithine et 0,8 à 1,9 mg d'EDTA par dose unitaire, et dans laquelle la formulation est une poudre fluide.

12. Formulation vétérinaire orale pour une utilisation dans un procédé de traitement de la diarrhée chez un porcelet, dans laquelle la formulation vétérinaire orale comprend une quantité de bromélaïne efficace contre la diarrhée et un émulsifiant en une quantité efficace pour améliorer la solubilité de ladite bromélaïne dans l'eau, et dans laquelle le porcelet est non sevré ou en cours de sevrage, et dans laquelle la formulation comprend la formulation vétérinaire orale selon la revendication 11 ou 12 en suspension dans l'eau.

13. Formulation vétérinaire orale pour l'utilisation selon l'une quelconque des revendications 10 à 12, dans laquelle le procédé comprend l'administration au porcelet d'une dose unique d'une quantité thérapeutiquement efficace de la formulation vétérinaire orale.
